# EUROPEAN PATENT SPECIFICATION

(11) **EP 3 500 265 B1**
(45) Date of publication and mention of the grant of the patent: **04.10.2023**
(21) Application number: 17844206.7
(22) Date of filing: 21.08.2017
(51) Int. Cl.: C07J 1/00, A61P 5/30, A61K 31/565, A61P 9/00, A61P 15/12, A61P 17/00

(54) **ESTROGEN RECEPTOR BETA SELECTIVE LIGANDS**
ÖSTROGENREZEPTOR-BETA-SELEKTIVE LIGANDEN
LIGANDS SÉLECTIFS DU RÉCEPTEUR BÊTA DES OESTROGÈNES

(30) Priority: 22.08.2016 US 201662378015 P; 22.08.2016 US 201662378014 P
(43) Date of publication of application: 26.06.2019
(73) Proprietor: Or-Genix Therapeutics, Inc., Worcester, MA 01605 (US)
(72) Inventor: SCHWARTZ, Yael, Worcester, MA 01605 (US); ABOLIN, Craig, Worcester, MA 01605 (US)
(74) Representative: Murgitroyd & Company
(86) International application number: PCT/US2017/047752
(87) International publication number: WO 2018/039110

(56) References cited:
- WO-A2-02/058634
- US-A1- 2004 110 732
- US-A1- 2006 074 060
- MICHIELS P J A ET AL: "Ligand-based NMR spectra demonstrate an additional phytoestrogen binding site for 17@b-hydroxysteroid dehydrogenase type 1", JOURNAL OF STEROID BIOCHEMISTRY AND MOLECULAR BIOLOGY, ELSEVIER SCIENCE LTD., OXFORD, GB, vol. 117, no. 4-5, 1 November 2009 (2009-11-01), pages 93-98, XP026719207, ISSN: 0960-0760, DOI: 10.1016/J.JSBMB.2009.07.004 [retrieved on 2009-07-23]
- TAPOLCSANYI P ET AL: "Synthesis and receptor-binding examination of 16-hydroxymethyl-3,17-estradiol stereoisomers", STEROIDS, ELSEVIER SCIENCE PUBLISHERS, NEW YORK, NY, US, vol. 67, no. 7, 1 June 2002 (2002-06-01), pages 671-678, XP004353736, ISSN: 0039-128X, DOI: 10.1016/S0039-128X(02)00020-X
- ALLAN ET AL.: 'Modification of Estrone at the 6, 16, and 17 Positions: Novel Potent Inhibitors of 17b-Hydroxysteroid Dehydrogenase Type 1' JOURNAL OF MEDICINAL CHEMISTRY vol. 49, 2006, pages 1325 - 1345, XP002684169
- LABAREE ET AL.: 'Estradiol-16alpha-carboxylic Acid Esters as Locally Active Estrogens' JOURNAL OF MEDICINAL CHEMISTRY vol. 44, 2001, pages 1802 - 1814, XP055468725

## Description

This application priority to US Provisional Patent Application Ser. Nos. 62/378,014 and 62/378,015, filed on August 22, 2016.

### FIELD OF THE INVENTION

The present invention relates generally to estrogen receptor β selective ligands and, in various embodiments, more specifically to 16β-substituted estradiol derivatives. The estrogen receptor β selective ligands can be formulated for topical administration for therapeutic and/or cosmeceutical uses.

### BACKGROUND

Estrogen based therapies (e.g., hormone replacement therapy, or HRT, oral contraceptives, and the like) are common and generally considered effective. However, most systemic estrogen based therapies have known side effects, including cancer and stroke. Estrogen therapy, directly and indirectly, affects a large number of organs and some of the outcomes associated with estrogen therapy are deleterious. In some cases, undesired effects can be mitigated by local rather than systemic administration. However, even topical estrogen can still enter the bloodstream and have systemic effects. Accordingly, there remains a need for improved, topical selective estrogen based therapies having reduced side effects and off target effects. WO-A-02/058634 discloses estradiol derivatives with 16α-carboxy-, 16α-carboxy-methyl and 16α-carboxy-ethyl substitutions having activity on the estrogen receptor. J. Steroid Biochem. Mol. Biol., Vol 117(4/5) pp 93-98 (2009) and J. Med. Chem., Vol 49(4) pp 1325-1345 (2006) disclose the two compounds disclaimed from the scope of the present invention as well as their activity in the inhibition of 17-hydroxysteroid dehydrogenase.

### SUMMARY OF THE INVENTION

The invention is based, at least in part, upon the discovery that certain synthetic 16β estradiols are selective estrogen receptor β (EKβ) agonists, and that these 16β estradiols are effective for topical (e.g., local) administration substantially without systemic delivery or side effects. Accordingly, the EKβ selective ligands can be used to selectively target tissue enriched in EKβ (versus α). For example, the compounds can be topically applied and used to produce estrogenic effects in skin, hair, blood vessels, and mucosa.

In various aspects and embodiments, the invention provides a compound according to Structure 1: 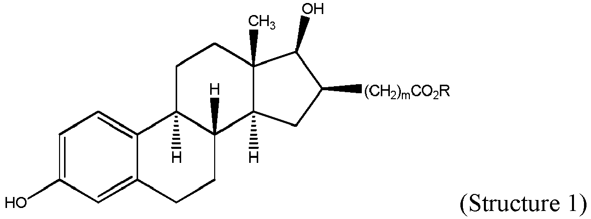 wherein m is 0, 1, or 2 and R is H, a C₁ to C₅ alkyl group, vinyl, CF₃, CH₂CH₂F, CH₂CHF₂, or CH₂CF₃, wherein the following compounds are excluded from the scope of Structure 1:

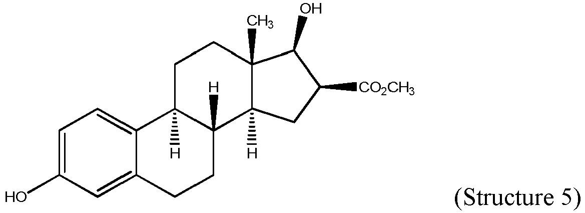

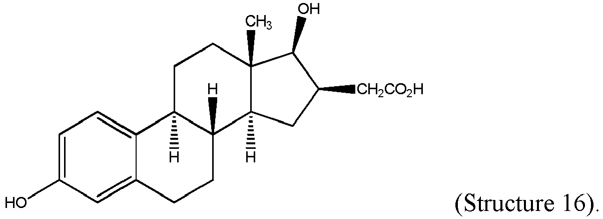

In various aspects and embodiments, the invention provides a composition comprising an effective amount of a compound according to the invention (e.g., any one or more of Structure 1-4, 6-15 and 17) and a physiologically acceptable carrier, diluent, or excipient, formulated for topical administration.

In various aspects and embodiments, the invention provides the compounds of the invention for use in a method comprising administering to a subject in need thereof an effective amount of a compound according to the invention (e.g., any one or more of Structures 1-4, 6-15 and 17) or a composition comprising thereof.

In various aspects and embodiments, the invention provides compounds or compositions of the invention for use in a method of treating a subject comprising administering a compound or composition of the invention to the subject in need thereof, thereby treating the subject.

In various aspects and embodiments, the invention provides the compounds of the invention for use as a selective agonist for estrogen receptor subtype ERβ.

As will be understood by those skilled in the art, any of the aspects and embodiments above can be combined with any one or more of the features below.

In various embodiments, m is 0 and R is methyl, ethyl, propyl, iso-propyl, butyl, iso-butyl, pentyl, neo-pentyl, vinyl, CF₃, CH₂CH₂F, CH₂CHF₂, or CH₂CF₃.

In various embodiments, m is 0 and R is methyl, ethyl, propyl, iso-propyl, butyl, iso-butyl, pentyl, neo-pentyl, vinyl, CF₃, CH₂CH₂F, CH₂CHF₂, or CH₂CF₃.

In various embodiments, m is 1 and R is a C1 or C3 to C5 alkyl group, vinyl, CF₃, CH₂CH₂F, CH₂CHF₂, or CH₂CF₃.

In various embodiments, m is 2.

In various embodiments, the compound is:

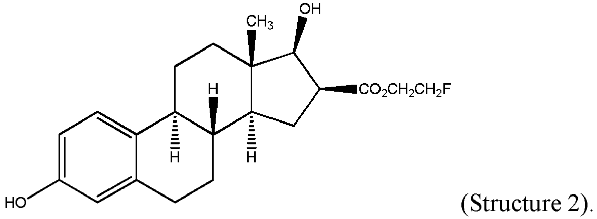

In various embodiments, the compound is:

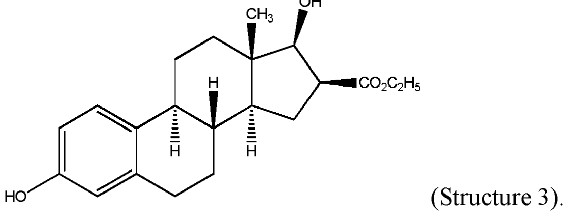

In various embodiments, the compound is:

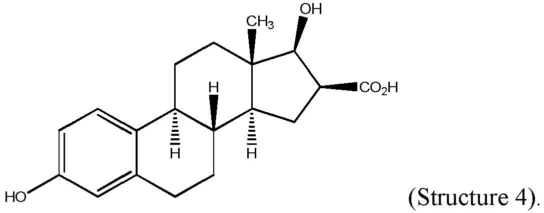

In various embodiments which do not form part of the present invention and are provided for illustrative purposes only, the compound is:

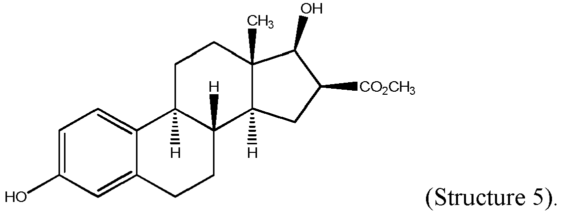

In various embodiments, the compound is:

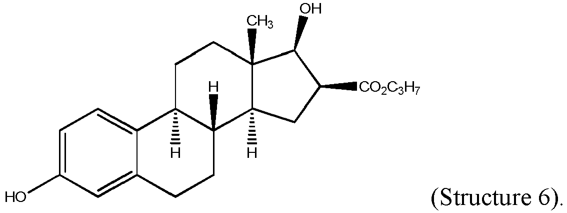

In various embodiments, the compound is:

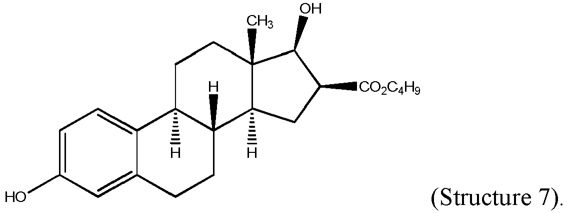

In various embodiments, the compound is:

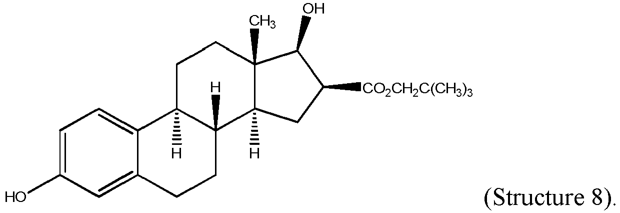

In various embodiments, the compound is:

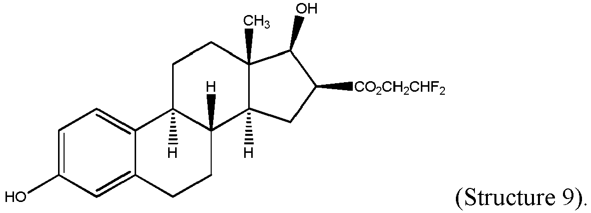

In various embodiments, the compound is:

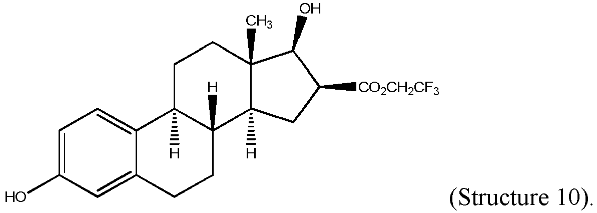

In various embodiments, the compound is:

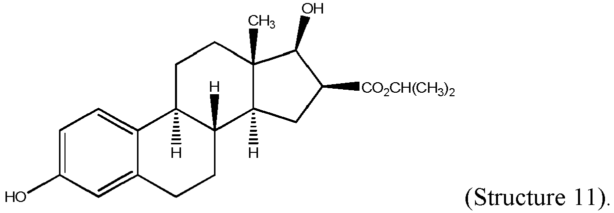

In various embodiments, the compound is:

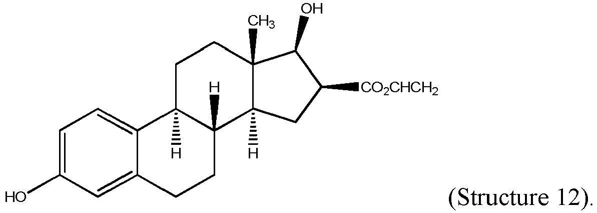

In various embodiments, the compound is:

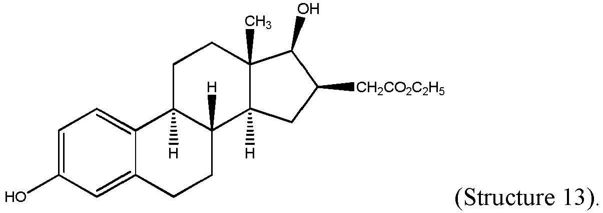

In various embodiments, the compound is:

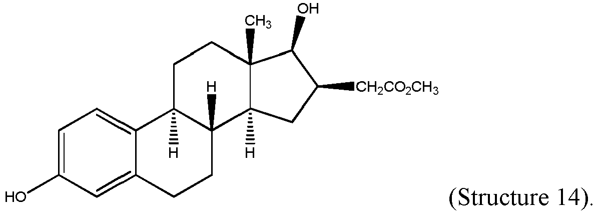

In various embodiments, the compound is:

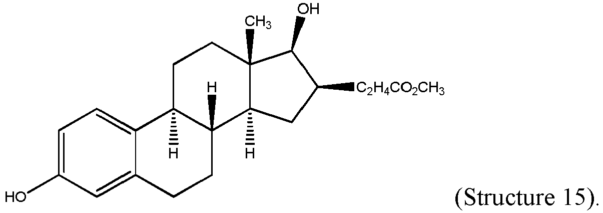

In various embodiments which do not form part of the present invention and are provided for illustrative purposes only, the compound is:

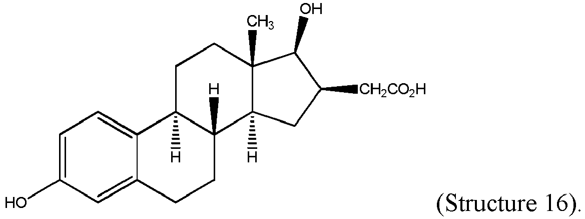

In various embodiments, the compound is:

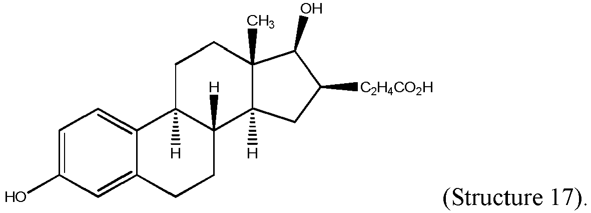

In certain embodiments, the compound does not include: m = 0 and R = H; m = 0 and R = CH₃; m = 1 and R = CH₃; and/or m = 1 and R = CH₂CH₃.

In some embodiments, the compound is not Structure 4, or 17 (wherein R = H). In certain other embodiments, the compound is not Structure 13.

In various embodiments, the compound is a 16β estradiol carboxylic acid ester.

In various embodiments, the compound is isolated (e.g., substantially enantiomerically pure). Hence, in certain embodiments, the composition includes only the isolated (e.g., substantially enantiomerically pure) compound as the active pharmaceutical ingredient. The composition can be substantially free of other estrogenic compounds, for example enantiomers, in particular 16α enantiomers.

In various embodiments, the compound is selective for EPβ over ERα. The ratio of the affinities for estrogen receptor subtypes (EPβ over ERα) can be at least about 2, 5, 10, 20, 30, 40, 50, 60, 70, 80, 90, 100, or 1000.

In various embodiments, the composition is used for and may specifically be formulated for topical administration to skin (e.g., such as to the face, scalp, neck, décolletage, chest, arms, hands, legs, and the like).

In certain embodiments, the composition is formulated for topical administration to a mucous membrane. In one embodiment, the composition is formulated for vaginal administration. In another embodiment, the composition is formulated for nasal administration. In yet another exemplary embodiment, the composition is formulated for rectal administration. In one embodiment, the composition is formulated for ocular administration.

In various embodiments, the subject to whom the compound or composition is administered is a mammal. For example, the subject can be a human. The subject can be a non-human primate. In various embodiments, the subject is an animal with estrogen receptors, preferably ERβ. The subject can be a female or male. In certain embodiments, the subject is a human female.

In certain embodiments, the subject has an estrogen deficiency or imbalance. The deficiency or imbalance can be related to aging, for example, in a menopausal or postmenopausal female or in a pre- or peri-menopausal female.

In certain embodiments, the compositions of the invention (including the various formulations) can be for prescription use and be prescribed by a doctor for a clinically recognized disease or condition associated with an estrogen deficiency or imbalance.
Alternatively, the compositions can be available over the counter (or prescribed by a doctor such as a dermatologist) for discretionary or cosmetic use.

In various embodiments, the administration is topical. The topical administration can be to skin or mucosa as described above.

In various embodiments, the compound acts substantially without systemic effects (e.g., acts at and/or proximally to the site of administration, but does not substantially enter circulation and/or effect tissue distally to the site of administration).

In various embodiments, the compound acts substantially without off target effects (e.g., effects the target tissue, but not other tissue).

In various embodiments, the treatment is cosmetic or cosmeceutical.

In various embodiments, the ERβ is selectively targeted by administering the compound to a subject.

In various embodiments, the subject has a disease or condition associated with an estrogen deficiency or imbalance.

In various embodiments, the estrogen deficiency or imbalance is due to unopposed androgens.

In various embodiments, the disease or condition relates to hair growth.

The invention also features a composition according to any of the aspects and embodiments above, for use as a medicament. In various embodiments, the medicament can be for topical administration and can substantially avoid systemic delivery of the compound.

### BRIEF DESCRIPTION OF THE DRAWINGS

**FIG. 1A** shows the results of a functional estrogen assay for 16β agonist Structure 15. **FIG. 1B** shows the results of a functional estrogen assay for 16α agonist Structure 15'.
**FIG. 2A** shows the results of a binding assay for 16β agonist Structure 3.
**FIG. 2B** shows the results of a binding assay for 16α agonist Structure 3'.
**FIG. 3A** shows the results of a functional estrogen assay for 16β agonist Structure 3.
**FIG. 3B** shows the results of a functional estrogen assay for 16α agonist Structure 3'.
**FIG. 4** shows an example synthesis of 16β agonist Structure 3 and its 16α analog Structure 3'.
**FIG. 5** shows an HPLC of 99.7% pure Structure 3.
**FIG. 6** shows an NMR of Structure 3.
**FIG. 7** shows an HPLC of 99.5% pure Structure 3'.
**FIG. 8** shows an NMR of Structure 3'.

### DETAILED DESCRIPTION

The invention is based, at least in part, upon the discovery that certain synthetic 16β estradiols are selective ERβ agonists, and that these 16β estradiols are effective for topical (e.g., local) administration substantially without systemic delivery or corresponding side effects. Accordingly, the ERβ selective ligands can be used to selectively target tissue enriched in ERβ (versus α). For example, the compounds can be topically applied and used to produce estrogenic effects in skin (e.g., scalp), hair, blood vessels, and mucosa.

Aging skin has been tied to estrogen deficiency in postmenopausal women (Draelos Z. D. Topical and oral estrogens revisited for antiaging purposes. Fertil Steril 84, 291-292 (2005); Thornton M. J. Estrogens and aging skin. Dermato-Endocrinology 5, 264-270 (2013)). Estrogen receptor β (ERβ) is the dominant estrogen receptor human in skin (Thornton M. J. et al. Estrogen receptor beta is the predominant oestrogen receptor in human scalp skin. Exp Dermatol 12, 181-190 (2003); Pelletier G. & Ren L. Localization of sex steroid receptors in human skin. Histol Histopathol 19, 629-636 (2004); Beier K., Ginez I. & Schaller H. Localization of steroid hormone receptors in the apocrine sweat glands of the human axilla. Histochem Cell Biol 123, 61-65 (2005)). The present invention provides ERβ-selective estrogenic ligands, which can be used, for example, to treat estrogen deficiency topically (e.g., in skin, mucous membranes, and the like) with minimal systemic impact. Accordingly, the present invention is distinguished from endogenous estrogenic ligands such as estradiol, estrone, and estriol (as well as most synthetic estrogens), which are non-specific with respect to Estrogen receptor alpha (ERα) and ERβ (Kuiper G. G. J. M. et al. Comparison of the Ligand Binding Specificity and Transcript Tissue Distribution of Estrogen Receptors α and β. Endocrinology 138, (1997)).

Chemical modifications at the C16 position of estradiol have shown that some 16α derivatives are estrogenic (Reiner G. C. A., Katzenellenbogen B. S., Bindal R. D. & Katzenellenbogen J. A. Biological Activity and Receptor Binding of a Strongly Interacting Estrogen in Human Breast Cancer Cells. Cancer Res 44, 2302-2308 (1984), Fevig T. L., Mao M. K. & Katzenellenbogen J. A. Estrogen Receptor Binding Tolerance of 16a-Substituted Estradiol Derivatives. Steroids 51, 471-497 (1988), Labaree D. C., Reynolds T. Y. & Hochberg R. B. Estradiol-16α-carboxylic Acid Esters as Locally Active Estrogens. JMed Chem 44, 1802-1814 (2001)). A series of estradiol-16α-alkyl esters intended for topical use appeared to show preferential binding to ERα (Labaree D. C., Reynolds T. Y. & Hochberg R. B. Estradiol-16α-carboxylic Acid Esters as Locally Active Estrogens. J Med Chem 44, 1802-1814 (2001)). ERα is poorly expressed in skin and ERα ligands have been implicated in breast and endometrial cancer (Liao X-H. et al. Estrogen receptor α mediates proliferation of breast cancer MCF-7 cells via a p21/PCNA/E2F1-dependent pathway. FEBS Journal 281, 927-942 (2013), Bender D., Buekers T. & Leslie K. K. Hormones and Receptors in Endometrial Cancer. Proceedings in Obstetrics and Gynecology 2, 1-25 (2011)).

Chemical modifications to the C16 of estradiol can also produce non-estrogenic compounds capable of inhibiting the enzyme 17β-hydroxysteroid dehydrogenase Type 1 (17β-HSD1) that converts estrone to estradiol and are proposed to treat estrogen-dependent cancers. A series of 16α derivatives of estradiol were found to inhibit 17β-HSD1. Others have produced 16β estradiol derivatives that also inhibit 17β-HSD (Allan G. M. et al. Modifications of Estrone at the 6, 16 and 17 positions: Novel Potent Inhibitors of 17β-Hydroxysteroid Dehydrogenase Type 1. J. Med. Chem 49, 1325-1345 (2006)). Estrogenic compounds according to the present invention with ERβ selectivity can be advantageously applied for topical application, without the drawbacks of ERα ligands.

Many estradiol derivatives at the C16 position (both 16alpha and 16beta) are neither estrogenic nor suitable for topical administration to treat local estrogen deficiency or imbalance. See e.g., Paquette L. A., Dahnke K., Doyon J., He W., Wyant K. & Friedrich D. Regioselective Conversion of Cycloalkanones to Vinyl Bromides with 1,2-Functionality Transposition. A General Strategem. J. Org. Chem 56, 6199-6205 (1991); and Allan G. M. et al. Modifications of Estrone at the 6, 16 and 17 positions: Novel Potent Inhibitors of 17β-Hydroxysteroid Dehydrogenase Type 1. J. Med. Chem 49, 1325-1345 (2006).

16β estradiol compounds according to the present invention are ERβ selective and/or have high EKβ activity. For example, 16β derivatives according to the invention were prepared and tested against their 16α counterparts for ER specificity. In one test, summarized in Example 1 below, 16β derivative Structure 15 was tested against is 16α counterpart, shown below as Structure 15':

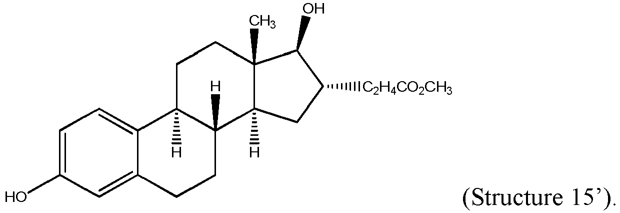

Both enantiomers are relatively weak estrogen agonists but were surprisingly specific for ERβ. The 16β derivative of the present invention (Structure 15) was more specific for ERβ than the corresponding 16α derivative.

In another test, summarized in Examples 2 and 3 below, 16β derivative Structure 3 was tested against its 16α counterpart, shown below as Structure 3':

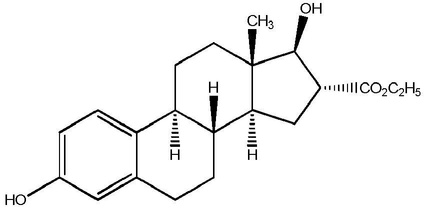

(Structure 3') for ER binding and functional activity. (The structures labeled as X' are corresponding 16α derivatives.) As expected, the 16α derivative was ERα-specific with respect to binding and functional activity. However, the 16β derivative (Structure 3) had comparable or marginally greater binding and functional ERα activity compared to the ERβ subtype.

Accordingly, the ERβ selective ligands can be used to selectively target tissue enriched in ERβ (versus ERα).

The various features of the invention including ERβ selective ligands, synthesis of ERβ selective ligands, topical formulations, treatment and administration, and illustrative examples are discussed, in turn, below.

### Estrogen Receptors

Estrogen receptors (ERs) are a group of proteins found inside and on cells that are activated by the hormone estrogen. The two classes of ER include nuclear estrogen receptors (ERα and ERβ), which are members of the nuclear receptor family of intracellular receptors, and membrane estrogen receptors (mERs) (GPER (GPR30), ER-X, and Gq-mER), which are mostly G protein-coupled receptors.

There are two different forms of the nuclear estrogen receptor, usually referred to as α and β, each encoded by a separate gene (ESR1 and ESR2, respectively). Hormone-activated estrogen receptors form dimers, and, since the two forms are coexpressed in many cell types, the receptors may form ERα (αα) or ERβ (ββ) homodimers or ERαβ (αβ) heterodimers. ERα and ERβ show significant overall sequence homology, and both are composed of five domains. It was widely assumed after the isolation and cloning of the human estrogen receptor (Greene G. L. et al. Sequence and Expression of Human Estrogen Receptor Complementary DNA. Science 231, 1150-1154 (1986); Green S. et al. Human oestrogen receptor cDNA: sequence, expression and homology to v-erb-A. Nature 320, 134-139 (1986)) that only one such receptor existed. Only ten years later, a novel gene coding for a different estrogen receptor was cloned (Mosselman S., Polman J. & Dijkema R. ERβ: identification and characterization of a novel human estrogen receptor. FEBS Letters 392, 49-53 (1996)). Both ERα and ERβ bind endogenous estrogen (17β-estradiol) with similar high affinity. However, ERα and ERβ can have different tissue distribution and function, as well as differing responses to other estrogenic ligands.

The present invention provides novel ERβ selective ligands, which can be used for topical application to treat estrogen deficiency and/or imbalance. The compounds can be particularly advantageous in treating skin, where ERβ is the major steroid receptor. In contrast to ERα and androgen receptors (AR), ERβ is highly expressed in epidermis, blood vessels and dermal fibroblasts. In the hair follicle, ERβ expression is localized to the nuclei of the outer root sheath, epithelial matrix and dermal papilla cells. This is in contrast to ERα and AR, which are only expressed in dermal papilla cells. There is strong nuclear expression of ERβ in the cells of the bulge, while neither ERα nor AR is expressed. In the sebaceous gland, ERβ is expressed in both basal and partially differentiated sebocytes. ERα exhibited a similar pattern of expression, while the AR is expressed in the basal and very early differentiated sebocytes. The wide distribution of ERβ in human skin suggests that estrogens may play an important role in the maintenance of skin and in the regulation of the pilosebaceous unit (Thornton M. J. et al. The Distribution of Estrogen Receptor β Is Distinct to That of Estrogen Receptor α and the Androgen Receptor in Human Skin and the Pilosebaceous Unit. J Inves Dermatol Symp Proc 8, 100-103 (2003)) The ERβ selective ligands according to the invention are useful for targeting these ERβ enriched tissues.

### Estrogen Receptor β Selective Ligands

In various aspects and embodiments, the invention provides ERβ selective ligands, specifically 16β estradiols. The ligands can be selective for ERβ over ERα in terms of binding and/or activity. The ligands can be effective for topical (e.g., local) administration substantially without systemic delivery and/or systemic effects.

In various aspects and embodiments, the 16β estradiols are more specific for the β-subtype of the estrogen receptor. In various embodiments, such 16β estradiols can stimulate hair growth. This is evidenced by the observation that, for example, during pregnancy, when estrogen levels are very high, women often experience hair growth due to prolongation of the anagen phase (the growth phase) aspect of the hair growth cycle. In contrast, the plummeting post-partum and menopausal estrogenic levels cause thinning and falling hair, sometimes resulting in clinically significant hair loss.

In various embodiments, the compound is selective for ERβ over ERα. The ratio of the affinities of estrogen receptor subtypes (EPβ over ERα) can be at least about 2, 5, 10, 20, 30, 40, 50, 60, 70, 80, 90, 100, or 1000. By way of comparison, the ratio of the affinities of estrogen receptor subtypes (EPβ over ERα) is about 1 for estradiol (J. Med. Chem. (2003) 46, 1886-1904).

In various aspects and embodiments, the invention provides a compound according to Structure 1: 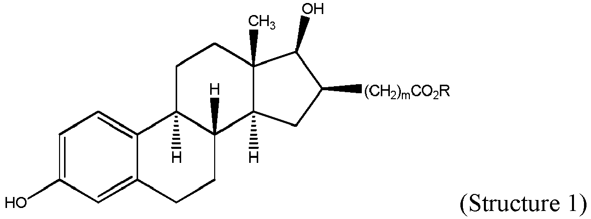 wherein m is 0, 1, or 2 and R is H, a C₁ to C₅ alkyl group, vinyl, CF₃, CH₂CH₂F, CH₂CHF₂, or CH₂CF₃, wherein the following compounds are excluded from the scope of Structure 1:

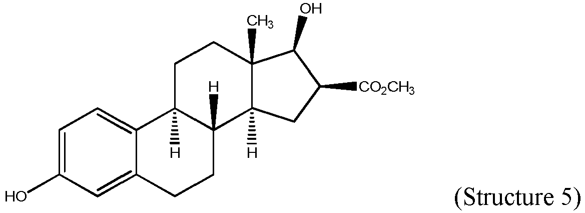

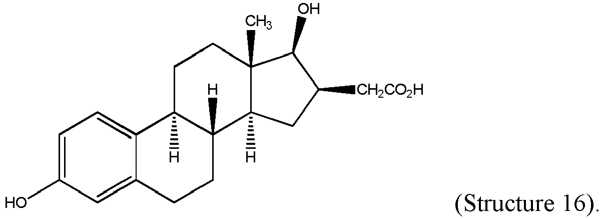

In various embodiments, m is 0 and R is methyl, ethyl, propyl, iso-propyl, butyl, iso-butyl, pentyl, neo-pentyl, vinyl, CF₃, CH₂CH₂F, CH₂CHF₂, or CH₂CF₃.

In various embodiments, m is 0 and R is ethyl, propyl, iso-propyl, butyl, iso-butyl, pentyl, neo-pentyl, vinyl, CF₃, CH₂CH₂F, CH₂CHF₂, or CH₂CF₃.

In various embodiments, m is 1 and R is a C₁ or C₃ to C₅ alkyl group, vinyl, CF₃, CH₂CH₂F, CH₂CHF₂, or CH₂CF₃.

In various embodiments, m is 2.

In various embodiments, the compound is:

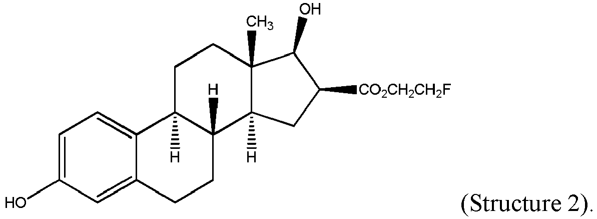

In various embodiments, the compound is:

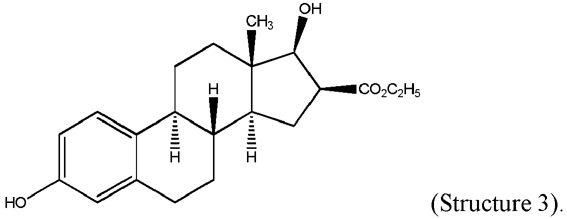

In various embodiments, the compound is:

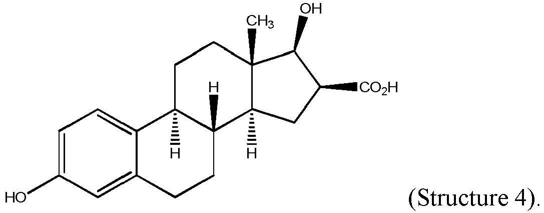

In various embodiments which do not form part of the present invention and are provided for illustrative purposes only, the compound is:

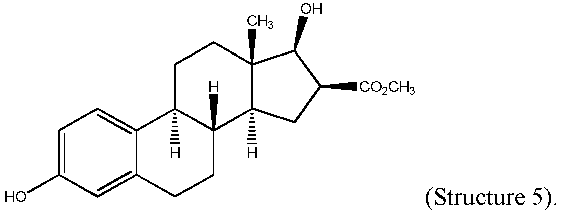

In various embodiments, the compound is:

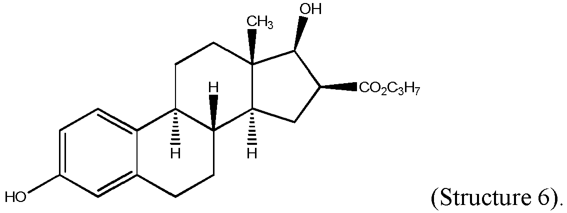

In various embodiments, the compound is:

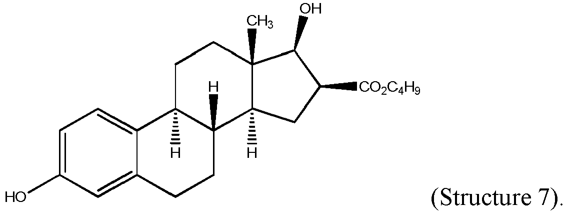

In various embodiments, the compound is:

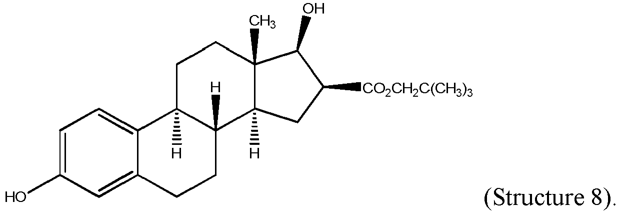

In various embodiments, the compound is:

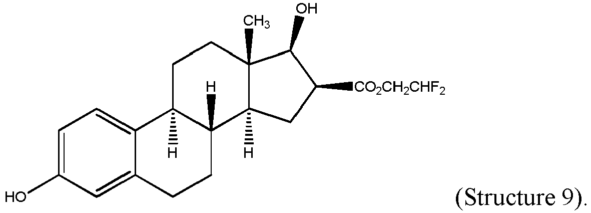

In various embodiments, the compound is:

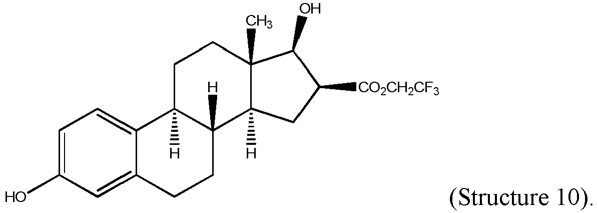

In various embodiments, the compound is:

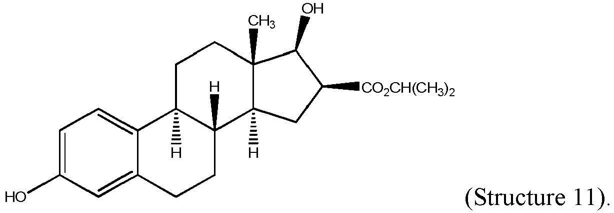

In various embodiments, the compound is:

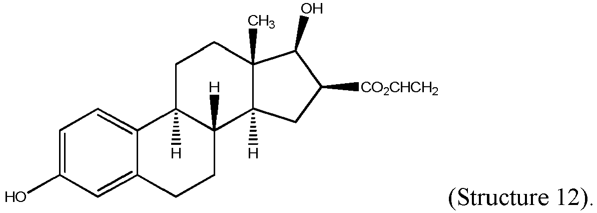

In various embodiments, the compound is:

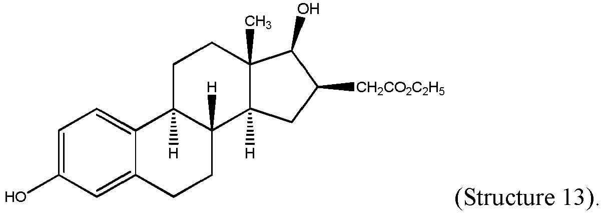

In various embodiments, the compound is:

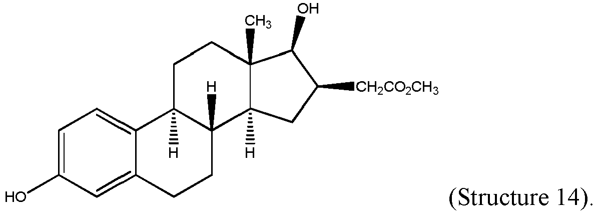

In various embodiments, the compound is:

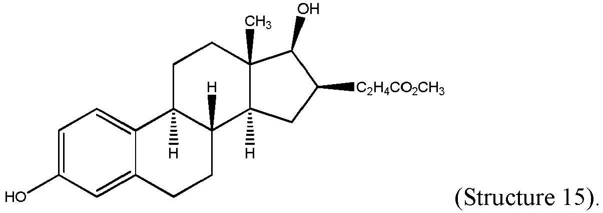

In various embodiments which do not form part of the present invention and are provided for illustrative purposes only, the compound is:

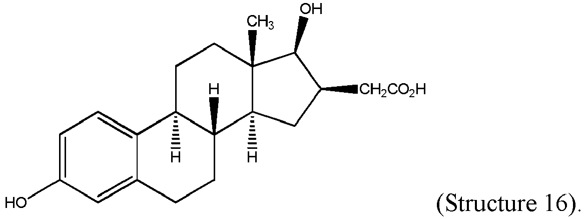

In various embodiments, the compound is:

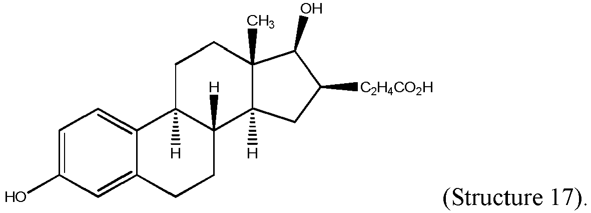

In various embodiments, the compound does not include: m = 0 and R = H; m = 0 and R = CH₃; m = 1 and R = CH₃; and/or m = 1 and R = CH₂CH₃.

In some embodiments, the compound is not Structure 4, 17 (wherein R = H). In certain other embodiments, the compound is not Structure 13. In various embodiments, the compound does not contain a carboxylic acid functional group attached to the C16 of the estradiol or estradiol derivative. In a preferred embodiment, the compound does not include: a) m=0 and R=H; b) m=1 and R=H; or c) m=2 and R=H.

In various embodiments, the compound is isolated (e.g., substantially enantiomerically pure). For example, the composition can contain at least 75%, 80%, 85%, 90%, 95%, 95%, 97% 98%, 99%, or more of enantiomerically pure 16β ligand(s). Hence, in some embodiments, the composition can include only the isolated (e.g., substantially enantiomerically pure) compound as the active pharmaceutical ingredient. The composition can be substantially free of other estrogenic compounds, for example enantiomers, in particular 16α enantiomers. As used herein, substantially free of other estrogenic compounds means the composition is free of all, or nearly all, estrogenic compounds other than the desired 16β ligand(s) of the invention. The composition can be 100% pure (e.g., within analytical limits), or less than 100% pure (e.g., a trace amount of other estrogenic compounds may be present, but which are not expected to have any physiologically relevant effect).

In various embodiments, the compound is a 16β estradiol carboxylic acid ester.

### Synthesis of Estrogen Receptor β Selective Ligands

A representative synthesis for Structure 3 is provided in Example 4 below. This method can be adapted for the synthesis of all compounds according to Structures 1-2 and 4-17 according to the ordinary skill in the art. For example, for Compounds 2, 3, 4, 5, 6, 7, 8, 9, 10, 11 and 12, one could use dimethyl carbonate in step one and the appropriate alcohol in step 4. For Compounds 13 and 14, one could use diethyl carbonate in step 1 and ethyl or methyl alcohol, respectively, in step 4. For Compound 15, one could use dipropyl carbonate in step 1 and methanol in step 4.

### Topical Formulations

In various aspects and embodiments, the invention provides a composition comprising an effective amount of a compound according to the invention (e.g., any one or more of Structures 1-4, 6-15 and 17) and a physiologically acceptable carrier, diluent, or excipient, formulated for topical administration. Examples of topical formulations include creams, gels, lotions, suppositories, patches, and the like.

The composition can include a compound or composition according to the invention and a physiologically acceptable excipient. As used herein, an excipient can be a natural or synthetic substance formulated alongside the active ingredient. Excipients can be included for the purpose of long-term stabilization, increasing volume (e.g., bulking agents, fillers, or diluents), or to confer an enhancement on the active ingredient in the final dosage form, such as facilitating absorption, reducing viscosity, or enhancing solubility. Excipients can also be useful manufacturing and distribution, for example, to aid in the handling of the active ingredient and/or to aid *in vitro* stability (e.g., by preventing denaturation or aggregation). As will be understood by those skilled in the art, appropriate excipient selection can depend upon various factors, including the route of administration, dosage form, and active ingredient(s).

The compositions can include an effective amount of the compound or composition according to the invention. As used herein, effective amount can be a concentration or amount that results in achieving a particular stated purpose, or more amount means an amount adequate to cause a change, for example in comparison to a placebo. Where the effective amount is a therapeutically effective amount, it can be an amount adequate for therapeutic use, for example and amount sufficient to prevent, diagnose, alleviate, treat, or cure a disease. An effective amount can be determined by methods known in the art. An effective amount can be determined empirically, for example by human clinical trials. Effective amounts can also be extrapolated from one animal (e.g., mouse, rat, monkey, pig, dog) for use in another animal (e.g., human), using conversion factors known in the art. See, e.g., Freireich et al., Cancer Chemother, Reports 50(4):219-244 (1966).

In various embodiments, the composition is formulated for topical administration to skin (e.g., such as to the face, scalp, neck, décolletage, chest, arms, hands, legs, and the like).

In various embodiments, the composition is formulated for topical administration to a mucous membrane. In one embodiment, the composition is formulated for vaginal administration. In one embodiment, the composition is formulated for nasal administration. In one embodiment, the composition is formulated for rectal administration. In one embodiment, the composition is formulated for ocular administration.

In various embodiments, the composition acts locally and/or substantially avoid systemic delivery of the compound according to the invention (e.g., any one or more of Structures 1-4, 6-15 and 17).

Topical formulations are known in the art and can be generally applied to the present invention by those skilled in the art. See, e.g., Cross S. E., Anderson C. & Roberts M. S. Topical penetration of commercial salicylic acid esters and salts using human isolated skin and clinical microdialysis studies. Br J Clin Pharmacol 46, 29-35 (1998); Neubert R. H. H. et al. Controlled Penetration of a Novel Dimeric Ceramide into and across the Stratum Corneum Using Microemulsions and Various Types of Semisolid Formulations. Skin Pharmacol Physiol 29, 130-134 (2016); Siddique M. I. et al. In-vivo dermal pharmacokinetics, efficacy, and safety of skin targeting nanoparticles for corticosteroid treatment of atopic dermatitis. Int J Pharmaceut 507, 72-82 (2016); and Wosicka H. & Cal K. Targeting to the hair follicles: Current status and potential. J Dermatolog Sci 57, 83-89 (2010).

Similarly, pharmaceutical and cosmeceutical formulations are known in the art and can be generally applied to the present invention by those skilled in the art. See, e.g., Damle M. & Mallya R. Development and Evaluation of a Novel Delivery System Containing Phytophospholipid Complex for Skin Aging. AAPS PharmSciTech 17, 607-617 (2016); and Draelos Z. D., Raab S., Yatskayer M., Chen N., Krol Y. & Oresajo C. A Method for Maintaining the Clinical Results of 4% Hydroquinone and 0.025% Tretinoin With a Cosmeceutical Formulation. J Drugs Dermatol 14, 386-390 (2015).

Furthermore, many additional approaches to topical formulation are known in the art for targeting specific tissues and can be readily applied to the compounds of the invention. See, e.g., Mosselman S., Polman J. & Dijkema R. ERβ: identification and characterization of a novel human estrogen receptor. FEBS Letters 392, 49-53 (1996); Green S. et al. Human oestrogen receptor cDNA: sequence, expression and homology to v-erb-A. Nature 320, 134-139 (1986); Greene G. L. et al. Sequence and Expression of Human Estrogen Receptor Complementary DNA. Science 231, 1150-1154 (1986); Pelletier G. & Ren L. Localization of sex steroid receptors in human skin. Histol Histopathol 19, 629-636 (2004). Kuiper G. G. J. M. et al. Comparison of the Ligand Binding Specificity and Transcript Tissue Distribution of Estrogen Receptors α and β. Endocrinology 138, (1997); Thornton M. J. et al. The Distribution of Estrogen Receptor β Is Distinct to That of Estrogen Receptor α and the Androgen Receptor in Human Skin and the Pilosebaceous Unit. J Inves Dermatol Symp Proc 8, 100-103 (2003).; Pelletier J. D. & Poirier D. Synthesis and Evaluation of Estradiol Derivatives With 16α-(Bromoalkylamide), 16α-(Bromoalkyl) or 16α-(Bromoalkylynyl) Side Chain as Inhibitors of 17β-Hydroxysteroid Dehydrogenase Type 1 without Estrogenic Activity. Bioorganic & Medicinal Chemistry 4, 1617-1628 (1996); Reiner G. C. A., Katzenellenbogen B. S., Bindal R. D. & Katzenellenbogen J. A. Biological Activity and Receptor Binding of a Strongly Interacting Estrogen in Human Breast Cancer Cells. Cancer Res 44, 2302-2308 (1984); Chang K. C. N., et al., Estrogen Receptor β is a Novel Therapeutic Target for Photoaging. Mol Phαrmacol 77, 744-750 (2010); Thornton M. J. et al. Oestrogen receptor beta is the predominant oestrogen receptor in human scalp skin. Exp Dermatol 12, 181-190 (2003); Thornton M. J. Estrogens and aging skin. Dermato-Endocrinology 5, 264-270 (2013); Bender D., Buekers T. & Leslie K. K. Hormones and Receptors in Endometrial Cancer. Proceedings in Obstetrics and Gynecology 2, 1-25 (2011); Allan G. M. et al. Modifications of Estrone at the 6, 16 and 17 positions: Novel Potent Inhibitors of 17β-Hydroxysteroid Dehydrogenase Type 1. J Med Chem 49, 1325-1345 (2006); and Ohnemus U., Uenalan M., Inzunza J., Gustafsson J-A. & Paus R. The Hair Folicle as an Estrogen Target and Source. Endocrine Reviews 27, 677-706 (2006).

### Treatment and Administration

In various aspects and embodiments, the invention also provides the compounds of the invention for use in a method comprising administering to a subject in need thereof an effective amount of a compound according to the invention (e.g., any one or more of Structures 1-4, 6-15 and 17) or a composition according to the invention.

In various embodiments, the subject is a mammal. For example, the subject can be a human. The subject can be a non-human primate. In various embodiments, the subject is an animal with estrogen receptors, preferably ERβ. The subject can be a female (or, in some cases, male). For example, in various embodiments, the subject is a human female.

In various embodiments, the subject has an estrogen deficiency (e.g., a replacement therapy) or imbalance (e.g., in the case of unopposed androgens). The deficiency or imbalance can be related to aging, for example, in a menopausal or post-menopausal human female. However, the deficiency or imbalance can be in a pre- or peri-menopausal human female.

In various embodiments, the compositions and/or formulations can be for prescription use. For example, the compositions and/or formulations can be prescribed by a doctor for a clinically recognized condition associated with an estrogen deficiency or imbalance.

In various embodiments, the compositions and formulations can be for non-prescription use. For example, the compositions and/or formulations can be available over the counter (or prescribed by a doctor such as a dermatologist) for discretionary or cosmetic use.

In various embodiments, the compound acts substantially without systemic effects (e.g., acts at and/or proximally to the site of administration, but does not substantially enter circulation and/or effect tissue distally to the site of administration). In various embodiments, the compound acts substantially without off target effects (e.g., effects the target tissue, but not other tissue). For example, the compound can act on skin at the site of administration, but not skin or other organs elsewhere.

The invention also features a composition according to any of the aspects and embodiments above, for use as a medicament. In various embodiments, the medicament can be for topical administration and can substantially avoid systemic delivery of the compound.

In various embodiments, the composition or compound can be used to treat conditions relating to hair growth. In one embodiment, the composition or compound can be used to promote hair growth. In another embodiment, the composition or compound can inhibit facial hair growth, for example, in post-menopausal female subjects.

General methods for treatment and administration are known in the art and can be generally applied to the present invention by those skilled in the art. See, e.g., the references cited in the *Topical Formulations* section above.

### EXAMPLES

### Example 1: Comparison of 16β agonist Structure 15 and its 16α analog Structure 15' in α functional assay for ERβ and ERα

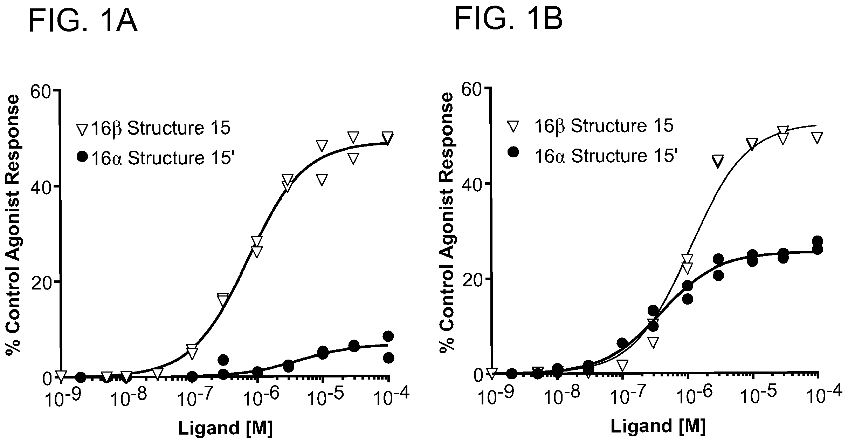

Structures 15 and 15' were tested in a functional assay for ERβ and ERα as described in Liu J. et al. A Homogeneous in Vitro Functional Assay for Estrogen Receptors: Coactivator Recruitment. Mol Endocrinology 17, 346-355 (2003). Briefly, a homogeneous *in vitro* functional assay for measuring ligand-dependent transactivation function of ERαa and ERβ was used to measure selectivity of structures 15 and 15' as agonists for the two classes of receptors. Briefly, agonist binding of Structures 15 and 15' were assessed by measuring Fluorescence Resonance Energy Transfer (FRET) between europium that is associated with ligand binding domains of ERα and ERβ, and allophycocyanine that is associated with coactivator peptide that is recruited to the LBD in response to agonist binding. FIG. 1A shows the results of a functional estrogen assay for 16β agonist Structure 15. FIG. 1B shows the results of a functional estrogen assay for 16α agonist Structure 15'.

As shown in FIG. 1A, 16β Structure 15 has strong ERβ activity, but relatively lower ERα activity. In contrast, as shown in FIG. 1B, 16α Structure 15' has similar ERβ activity, but also relatively higher ERα activity compared to 16β Structure 15. Therefore, Structure 15 acts as a selective ERβ binder and has markedly higher selectivity than its 16α analog Structure 15'. Therefore, receptor binding can predict functional activity for the compounds tested. For Compound 15, the EC50 is ~10 µM for ERβ and >100µM for ERα. Thus, Compound 15 has a >10-fold selectivity for the ERβ. For Compound 15' a ~2-fold selectivity for ERβ was estimated.

### Example 2: Binding Assay comparison of 16β agonist Structure 3 and its 16α analog Structure 3' in α binding assay for ERβ and ERα

Structures 3 and 3' were tested in a binding assay for ERβ and ERα as described in Obourn J. D., Koszewski N. J. & Notides A. C. Hormone- and DNA-Binding Mechanisms of the Recombinant Human Estrogen Receptor. Biochemistry 32, 6229-6236

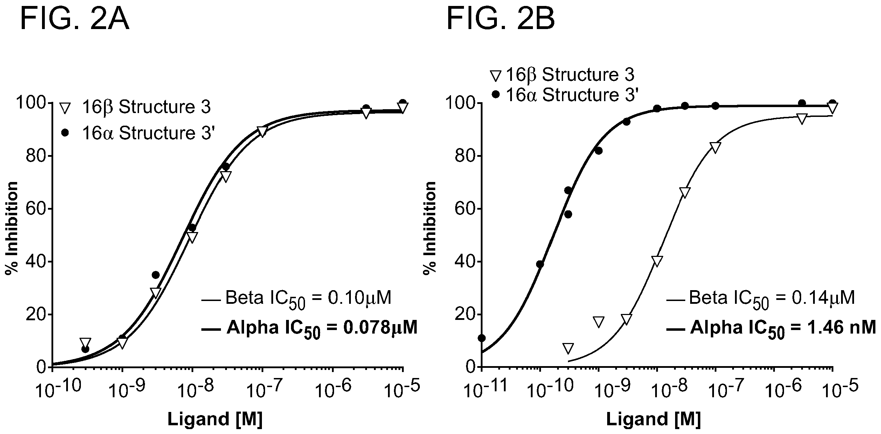

(1993). Briefly, agonist binding of Structure 3 and Structure 3' to ERα and ERβ were assessed by Scatchard analysis measuring inhibition of [³H] estradiol binding to hER by Structure 3 and 3'. FIG. 2A shows the results of a binding assay for 16β agonist Structure 3. FIG. 2B shows the results of a binding assay for 16α agonist Structure 3'. 16β Structure 3 binds ERβ and ERα with similar affinity (IC50 of 0.10 µM and 0.078 µM, respectively). In contrast 16α Structure 3' binds ERβ and ERα with affinity that differs by several orders of magnitude (IC₅₀ of 0.14 µM and 1.46 nM, respectively).

16β Structure 3 binds ERβ and ERα with similar affinity (IC₅₀ of 0.10 µM and 0.078 µM, respectively). In contrast 16α Structure 3' binds ERβ and ERα with affinity that differs by several orders of magnitude (IC50 of 0.14 µM and 1.46 nM, respectively). Therefore, Structure 3 acts as a selective ERβ binder and has markedly different ERβ binding properties than its 16α analog Structure 3'. These results indicate that both ER binding affinity and ER binding selectivity is sensitive to the stereochemistry at C16 of estradiol.

### Example 3: Function Assay comparison of 16β agonist Structure 3 and its 16α analog (Structure 3') in α functional assay for ERβ and ERα

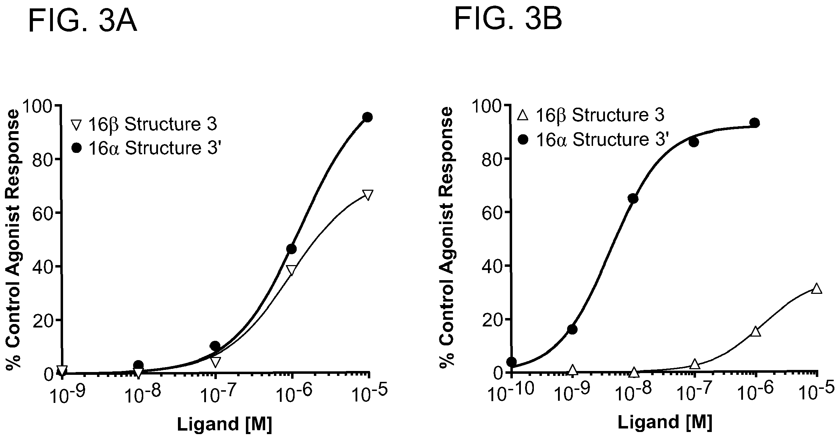

Structures 3 and 3' were tested in a functional assay for ERβ and ERα as described in Hilal T., Puetter V., Otto C. Parczyk K. & Bader B. A Dual Estrogen Receptor TR-FRET Assay for Simultaneous Measurement of Steroid Site Binding and Coactivator Recruitment. J Biomolec Screening 15, 268-278 (2010). Briefly, agonist binding of Structures 3 and 3' to ERα and ERβ were assessed by a dual estrogen receptor TR-FRET assay. FIG. 3A shows the results of a functional estrogen assay for 16β agonist Structure 3. FIG. 3B shows the results of a functional estrogen assay for 16α agonist Structure 3'.

16β Structure 3 has similar ERβ and ERα functional activity (EC50 of 3.0 µM and 1.08 µM, respectively). In contrast 16α Structure 3' has high ERα activity with minimal ERβ activity (EC50 of 5.92 nM and > 10 µM, respectively). Therefore, Structure 3 acts as a non-selective ER binder with markedly different binding properties than its 16α analog Structure 3'. The topical use of a non-selective ER agonist can mimic the nonselective effects of endogenous estrogens.

### Example 4: Synthesis of 16β agonist Structure 3

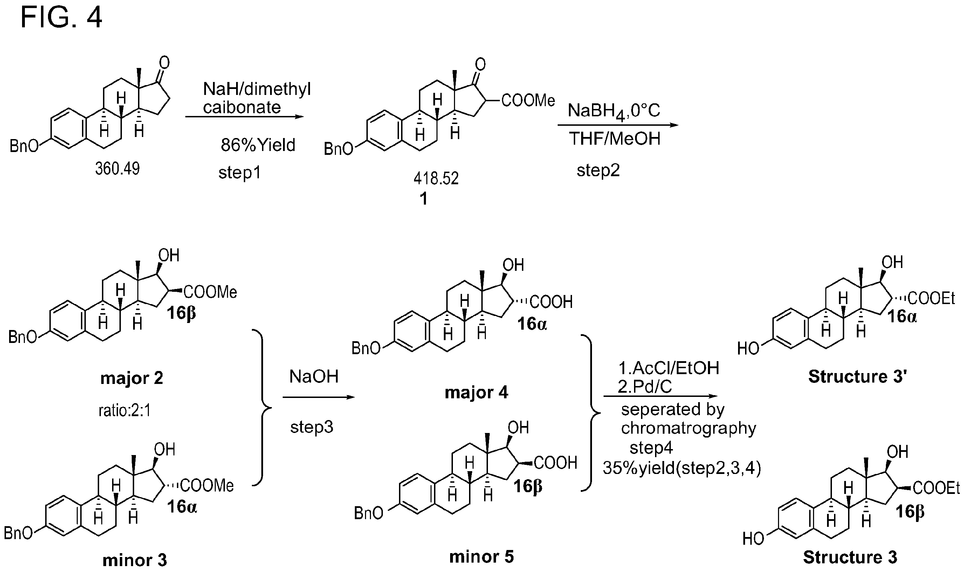

As shown in **FIG. 4****,** a 4-step process was performed at 10 g scale to produce Structure 3 and Structure 3'. First, 3-O-benzyl-estrone was reacted with dimethyl carbonate in the presence of NaH and in THF to give intermediate 1 in 86% yield after crystallization. Second, intermediate 1 was reduced by NaBH₄ to give diastereomeric mixtures of 2 and 3 (ratio 2/1), which are converted to intermediates 4 and 5 upon saponification. Third, after treatment with AcCl/MeOH followed by hydrogenation, the mixture of Structure 3 and Structure 3' was obtained. The mixture was separated by flash column chromatography.

### Step 1 (intermediate 1 in FIG. 4):

To a suspension of NaH (16.7 g, 0.348 mol) in THF (200 mL), dimethyl carbonate (26.1 g, 0. 290 mol, 2.5 eq) was added. The mixture was heated to reflux under N₂ protection. A solution of 3-O-benzylestrone (41.8 g, 0.116 mol) in THF (200 mL) was added over 1 h. After 4 h refluxing, the resulting reaction mixture was cooled to 0 °C, acidified with 3 M AcOH (200 mL), poured into 25% brine (1 L) and extracted with DCM (600 mL). The organic layer was dried over Na₂SO₄ (50 g) and concentrated in vacuum to give 64 g crude wet solid, which was crystallized in MeOH (400 mL) and dried under vacuum at 45 °C to give beige solid, intermediate 1, 41.7 g 99.5A% by LCMS (AP2277-10-1-3-LCMS, 13.9 min), 86% yield.

### Step 2 (mixture of intermediates 2 and 3 in FIG. 4):

To a suspension of intermediate 1 (10 g, 23.88 mmol) in a mixture of THF/ MeOH (180 mL/ 20 mL) at 0 °C, NaBH₄ (723 mg, 19.1 mmol, 0.8 eq) was added in portions over 20 min. After 1 h at 0 °C, the reaction mixture was acidified to pH =1-2 with 2 N HCl (15 mL), diluted with H2O (50 mL), extracted with EtOAc (200 mL). The organic layer was washed with brine (200 mL) and concentrated to give brown solid, 11.5 g over weight), which was used in the next saponification step without any further treatment.

### Step 3 (mixture of intermediates 4 and 5 in FIG. 4):

To a solution of the crude mixture of intermediates 2 and 3 (11.5 g, 27.3 mmol) in THF/MeOH (60 mL/60 mL), aq. NaOH (5.5 g, 136.7 mmol in 23 mL H2O) was added. After 2 h at 25-30 °C (LCMS indicated that 16α acid was 71.5A% and 16β acid was 18.6A%), the reaction mixture was cooled to 0-10 °C, acidified to pH 1-2 with 6 M HCl (40 mL), extracted with EtOAc (150 mL X 2). The combined organic extractions were washed with H₂O (200 mL) and 25% brine (200 mL), and concentrated to give crude solid, 12.5 g (over weight), which was used in the next step without any further treatment.

### Step 4 (Esterification and De-protection in FIG. 4):

To a solution of the crude mixture of intermediates 4 and 5 (12.5 g, 30.7 mmol) in EtOH (250 mL) at RT, AcCl (3.6 g, 46.1 mmol, 1.5 eq) was added. The solution was warmed to reflux for 20 h and then cooled to RT. 5% Pd/C (57.8 g) was added. The suspension was stirred under 1 atm H₂ at RT (25-30 °C) for 24 h. The solid was filtered off and the filtrate was neutralized with saturated NaHCO₃ (5 mL), diluted with H₂O (45 mL) and extracted with DCM (200 mL X 2). The combined organic extractions were washed with brine (200 mL) and concentrated to give 11 g viscous brown solid, which was purified by flash chromatography to give an off-white solid, Structure 3', 2.9 g, 99.5% pure by HPLC, 35% yield; and a white solid, 1.5 g, mixture of 16α (8.4%) and 16β isomer (86.7%), which was purified again by flash chromatography to give white solid, 200 mg, Structure 3, 99.7% pure by HPLC.

**Table 1. Characterization of reaction products**

| Product | Structure | Purity | 17-*CH* (*δ*ppm, *J*) | ¹H-NMR data |
|---|---|---|---|---|
| 16β-isomer (Structure 3) | 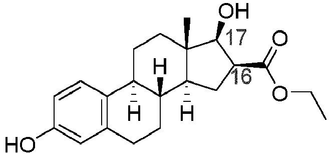 | 99.7% FIG. 5 | 3.83 (dd, *J* = 10.49, 5.25 Hz, 1H) | FIG. 6 |
| 16α-isomer (Structure 3') | 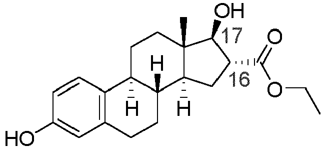 | 99.5% FIG. 7 | 3.70 (dd, *J* = 7.58, 5.75 Hz, 1H) | FIG. 8 |

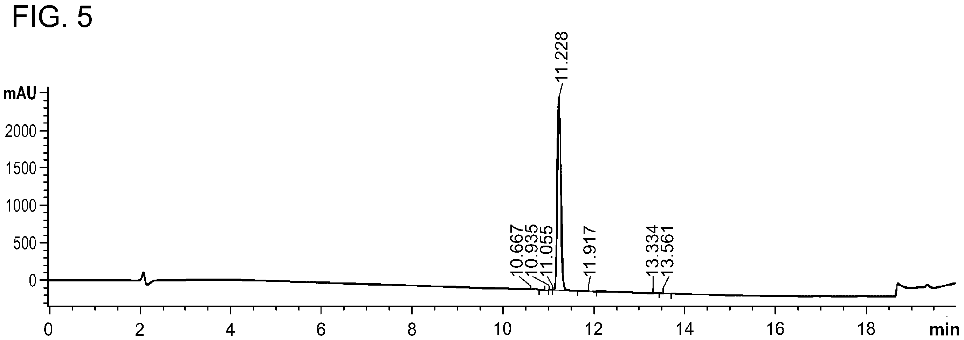

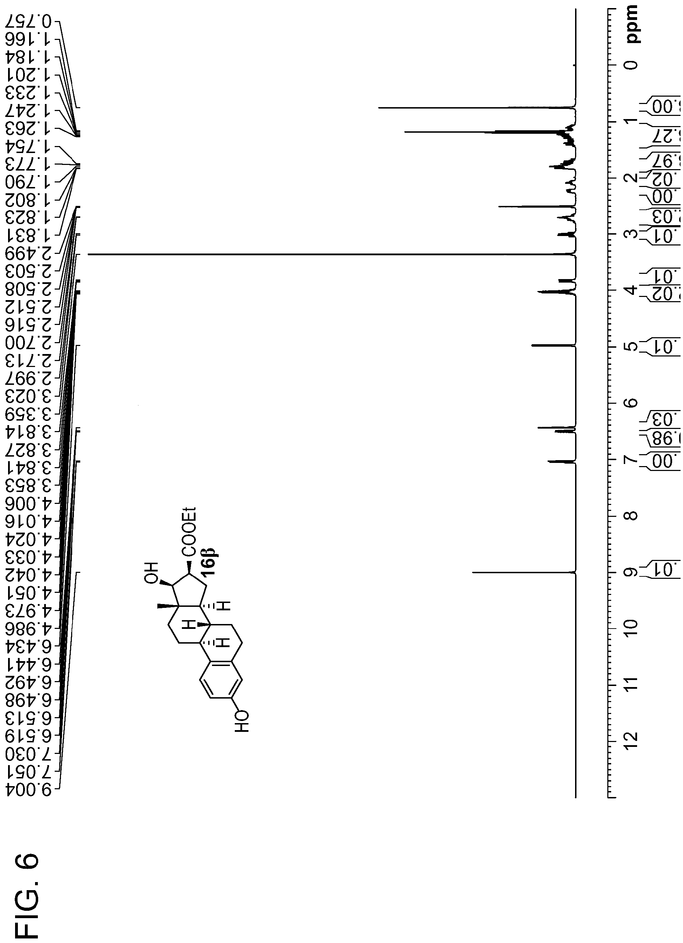

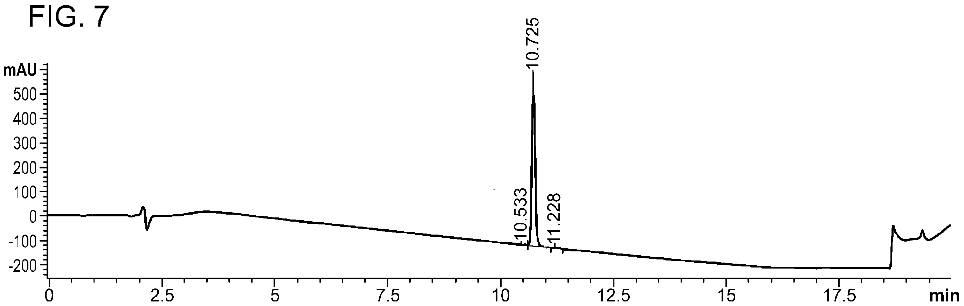

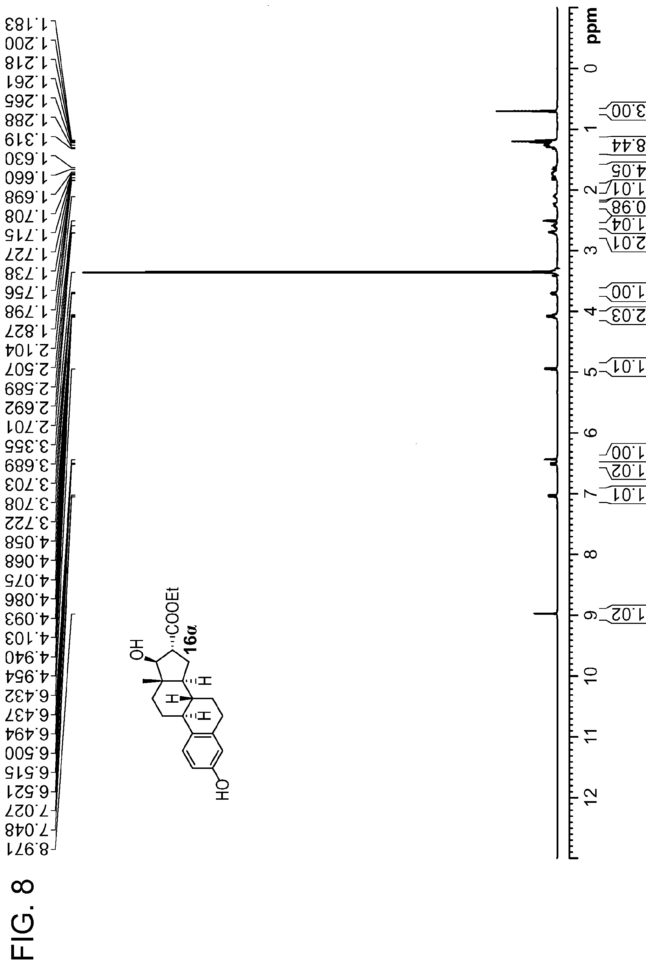

FIG. 5 shows an HPLC of 99.7% pure Structure 3. FIG. 6 shows an NMR of Structure 3. FIG. 7 shows an HPLC of 99.5% pure Structure 3'. FIG. 8 shows an NMR of Structure 3'.

### Example 5: Formulation of a topical cream comprising 16β agonist Structure 1

The composition is formulated into topical cream using any or all of the following ingredients: water; oil; emulsifier; thickening agent; other additives such as preservatives and perfumes; any one or more of 16β Structures 1-4, 6-15 and 17.

The topical cream formulation can either be in the form of an oil in water or water in oil emulsions. An effective amount of any one or more of 16β Structures 1-4, 6-15 and 17 is dissolved or dispersed in a cream base.

The resultant topical cream formulation is applied topically to an area of a subject's skin or mucous membrane.

### Example 6: Formulation of a topical ointment comprising 16β agonist Structure 1

The composition is formulated into a topical ointment using any or all of the following ingredients: hydrocarbon bases (e.g. hard paraffin, soft paraffin, microcrystalline wax and ceresine); absorption bases such as wool fat and/or beeswax; water-soluble bases such as macrogols 200, 300, and/or 400; emulsifying bases (e.g., emulsifying wax and/or cetrimide; vegetable oils such as olive oil, coconut oil, almond oil, and/or peanut oil); any one or more of 16β Structures 1-4, 6-15 and 17.

The topical ointment can be applied topically to an area of a subject's skin or mucous membrane.

An effective amount of any one or more of 16β Structures 1-4, 6-15 and 17 is dissolved or dispersed in the ointment base.

## Claims

1. A compound according to Structure 1: 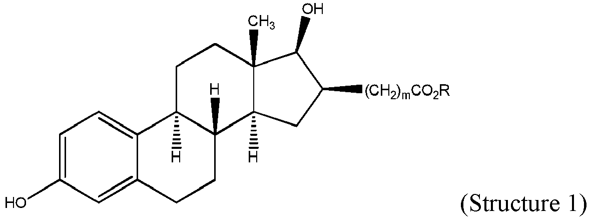 wherein m is 0, 1, or 2 and R is H, a C1 to C5 alkyl group, vinyl, CF₃, CH₂CH₂F, CH₂CHF₂, or CH₂CF₃, wherein the following compounds are excluded from the scope of Structure 1:

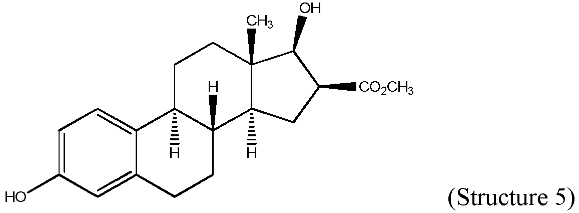

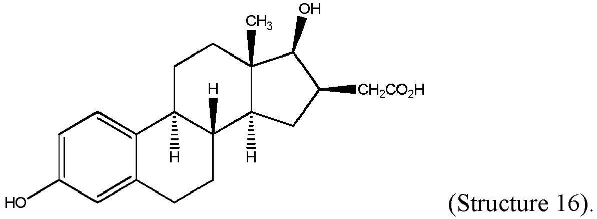

2. The compound of claim 1, wherein m is 0 and R is methyl, ethyl, propyl, iso-propyl, butyl, iso-butyl, pentyl, neo-pentyl, vinyl, CF₃, CH₂CH₂F, CH₂CHF₂, or CH₂CF₃.

3. The compound of claim 1, selected from the group consisting of:

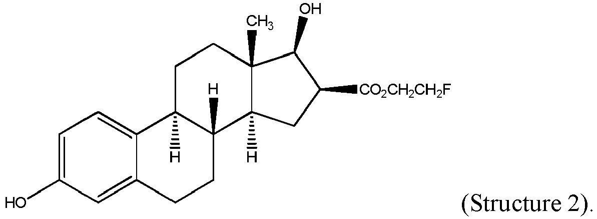

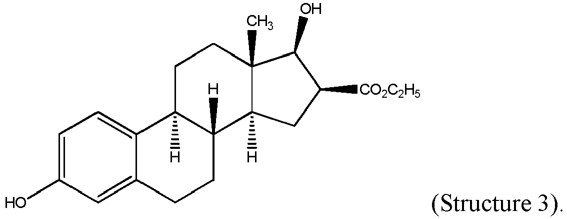

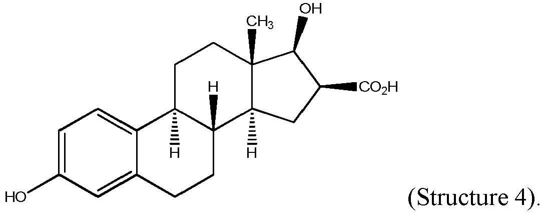

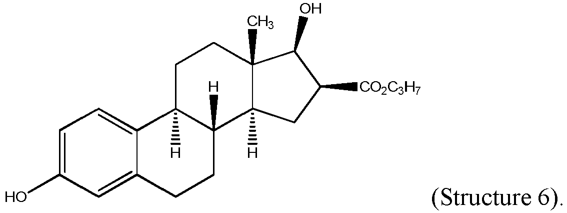

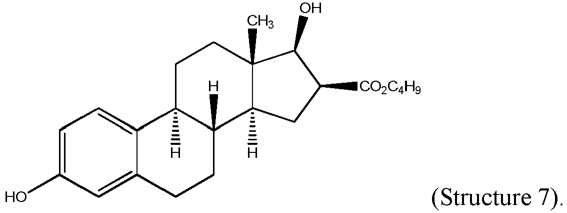

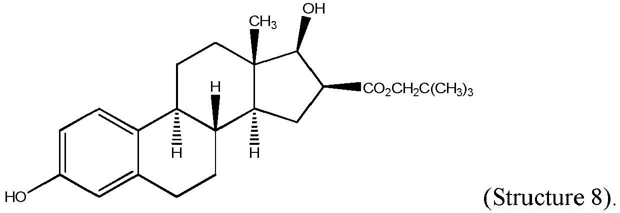

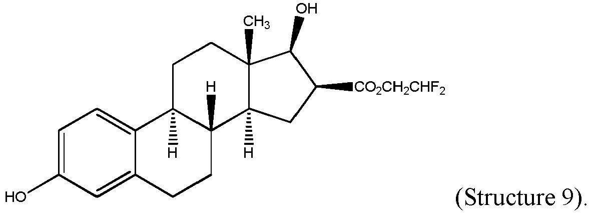

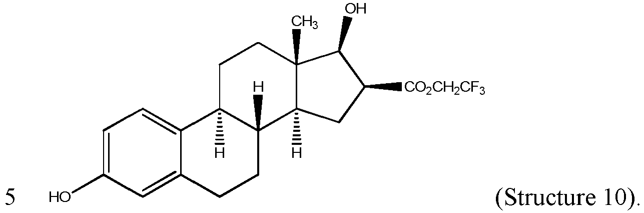

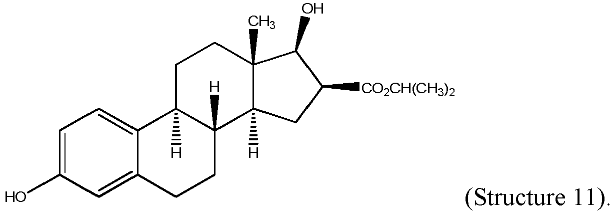

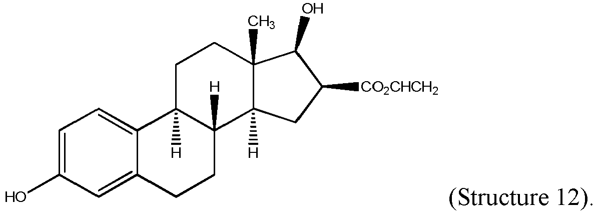

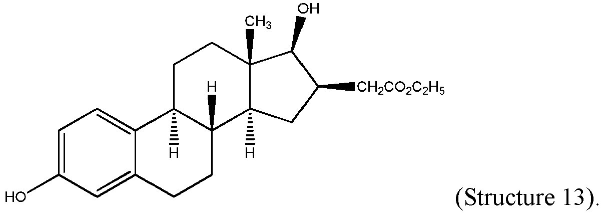

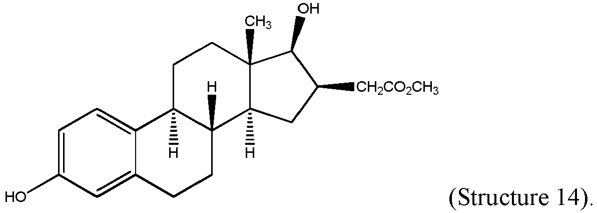

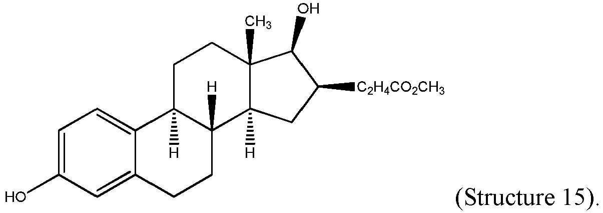

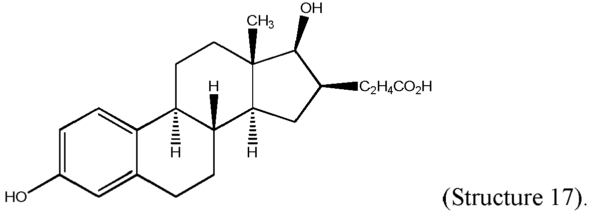

4. The compound of claim 1, wherein the compound is not a) Structure 4, or 17 (wherein R = H) or b) Structure 13 wherein these compounds are as defined in claim 3.

5. The compound of claim 1, wherein the compound is a 16β estradiol carboxylic acid ester.

6. A composition comprising a substantially enantiomerically pure compound according to any one of claims 1-5.

7. A composition comprising a compound according to any one of claims 1-5, which is substantially free of other estrogenic compounds.

8. A composition comprising an effective amount of a compound according to any one of claims 1-5 and a physiologically acceptable carrier, diluent, or excipient, formulated for topical administration.

9. The composition of claim 8, wherein the composition is formulated for topical administration to skin.

10. The composition of claim 8, wherein the composition is formulated for topical administration to a mucous membrane.

11. The composition of claim 8, comprising a substantially enantiomerically pure compound according to any one of claims 1-5.

12. The composition of claim 8, wherein the composition is substantially free of other estrogenic compounds.

13. The composition of any of claims 6 to 12, wherein the composition is a cosmetic or cosmeceutical.

14. The compound of any of claims 1 to 5 or composition of any of claims 6 to 13 for use as a medicament.

15. The compound or composition of claim 14, wherein the medicament is for use in the treatment of a subject having a disease or condition associated with an estrogen deficiency or imbalance.

16. The compound or composition of claim 15, wherein the estrogen deficiency or imbalance is due to unopposed androgens.

17. The compound or composition of claim 15, wherein the disease or condition relates to hair growth.

18. The compound or composition of claim 17, wherein the subject is a human female.

19. The compound or composition of claim 18, wherein the human female is post-menopausal.

20. The compound or composition of claim 18, wherein the human female is pre- or peri-menopausal.

## Patentansprüche

1. Eine Verbindung gemäß der Struktur 1: 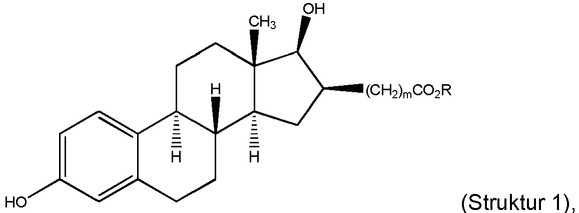 wobei m 0, 1 oder 2 ist und R H, eine C1- bis C5-Alkylgruppe, Vinyl, CF₃, CH₂CH₂F, CH₂CHF₂ oder CH₂CF₃ ist, wobei die folgenden Verbindungen aus dem Umfang von Struktur 1 ausgeschlossen sind:

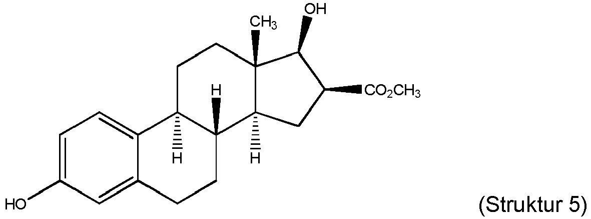

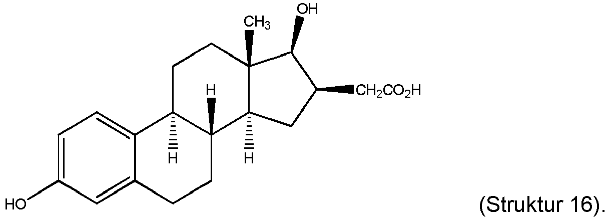

2. Verbindung gemäß Anspruch 1, wobei m 0 ist und R Methyl, Ethyl, Propyl, Isopropyl, Butyl, Isobutyl, Pentyl, Neopentyl, Vinyl, CF₃, CH₂CH₂F, CH₂CHF₂ oder CH₂CF₃ ist.

3. Verbindung gemäß Anspruch 1, die aus der Gruppe ausgewählt ist, die aus Folgendem besteht:

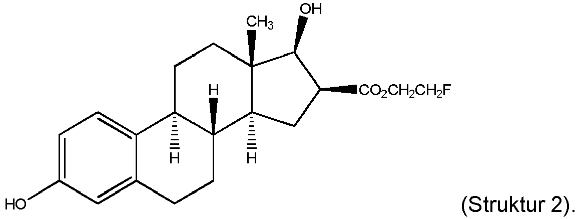

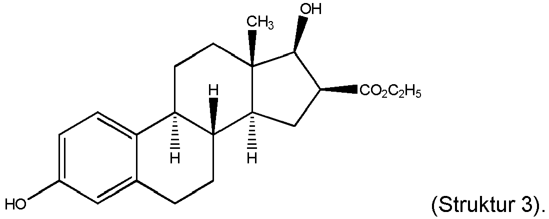

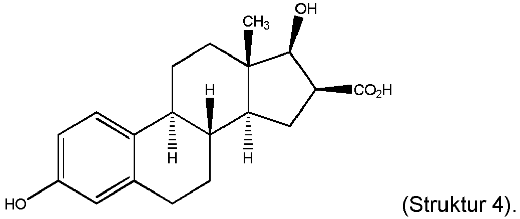

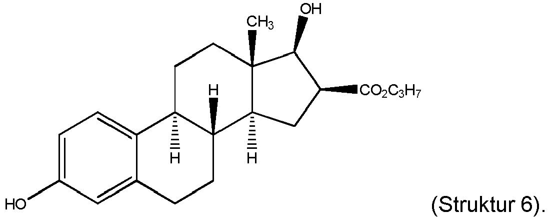

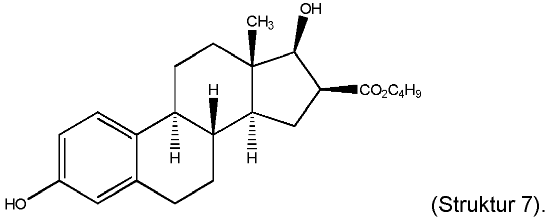

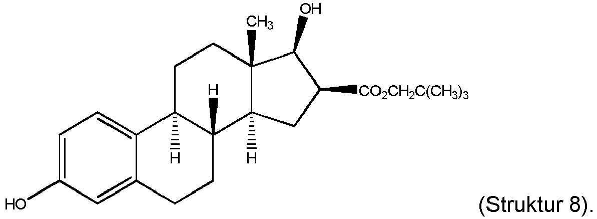

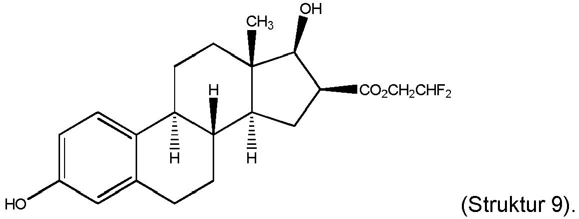

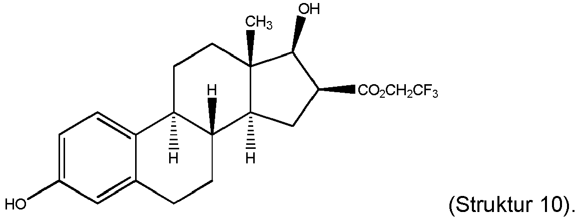

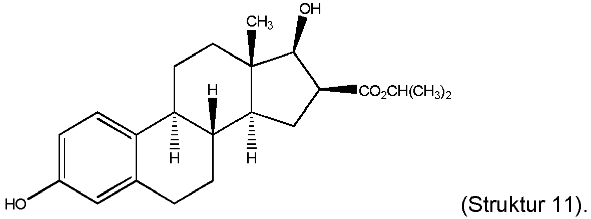

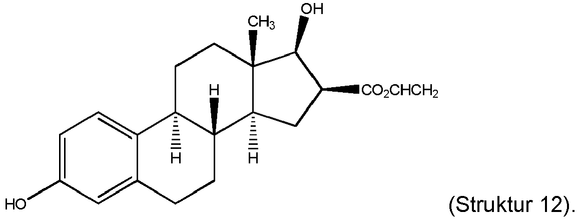

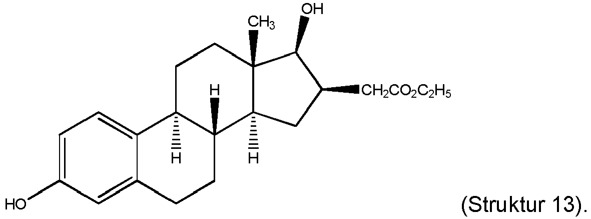

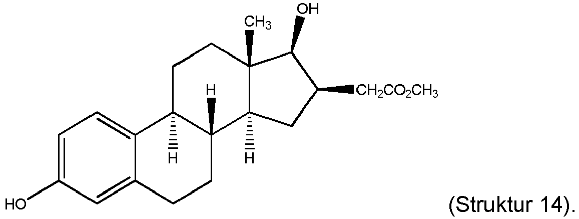

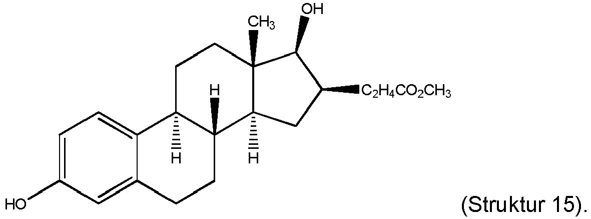

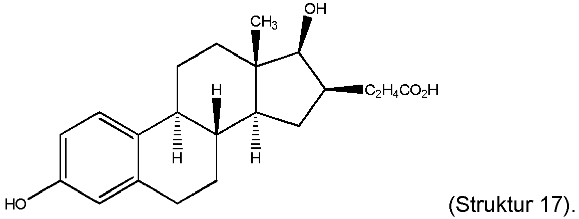

4. Verbindung gemäß Anspruch 1, wobei die Verbindung nicht a) Struktur 4 oder 17 (wobei R = H) oder b) Struktur 13 ist, wobei diese Verbindungen wie in Anspruch 3 definiert sind.

5. Verbindung gemäß Anspruch 1, wobei die Verbindung ein 16β-Estradiolcarbonsäureester ist.

6. Eine Zusammensetzung, die eine im Wesentlichen enantiomerreine Verbindung gemäß einem der Ansprüche 1-5 beinhaltet.

7. Eine Zusammensetzung, die eine Verbindung gemäß einem der Ansprüche 1-5 beinhaltet, die im Wesentlichen frei von anderen östrogenen Verbindungen ist.

8. Eine Zusammensetzung, die eine wirksame Menge einer Verbindung gemäß einem der Ansprüche 1-5 und einen physiologisch annehmbaren Träger, ein physiologisch annehmbares Verdünnungsmittel oder einen physiologisch annehmbaren Hilfsstoff beinhaltet, formuliert für die topische Verabreichung.

9. Zusammensetzung gemäß Anspruch 8, wobei die Zusammensetzung für die topische Verabreichung auf die Haut formuliert ist.

10. Zusammensetzung gemäß Anspruch 8, wobei die Zusammensetzung für die topische Verabreichung auf eine Schleimhaut formuliert ist.

11. Zusammensetzung gemäß Anspruch 8, die eine im Wesentlichen enantiomerreine Verbindung gemäß einem der Ansprüche 1-5 beinhaltet.

12. Zusammensetzung gemäß Anspruch 8, wobei die Zusammensetzung im Wesentlichen frei von anderen östrogenen Verbindungen ist.

13. Zusammensetzung gemäß einem der Ansprüche 6 bis 12, wobei die Zusammensetzung ein Kosmetikum oder Kosmezeutikum ist.

14. Verbindung gemäß einem der Ansprüche 1 bis 5 oder Zusammensetzung gemäß einem der Ansprüche 6 bis 13 zur Verwendung als Medikament.

15. Verbindung oder Zusammensetzung gemäß Anspruch 14, wobei das Medikament zur Verwendung bei der Behandlung eines Individuums mit einer Krankheit oder einem Zustand, die/der mit einem Östrogenmangel oder Östrogenungleichgewicht assoziiert ist, bestimmt ist.

16. Verbindung oder Zusammensetzung gemäß Anspruch 15, wobei der Östrogenmangel oder das Östrogenungleichgewicht auf nicht ausgeglichene Androgene zurückzuführen ist.

17. Verbindung oder Zusammensetzung zur Verwendung gemäß Anspruch 15, wobei sich die Krankheit oder der Zustand auf das Haarwachstum bezieht.

18. Verbindung oder Zusammensetzung gemäß Anspruch 17, wobei das Individuum eine menschliche Frau ist.

19. Verbindung oder Zusammensetzung gemäß Anspruch 18, wobei die menschliche Frau in der Postmenopause ist.

20. Verbindung oder Zusammensetzung gemäß Anspruch 18, wobei die menschliche Frau in der Prä- oder Perimenopause ist.

## Revendications

1. Un composé selon la Structure 1 : 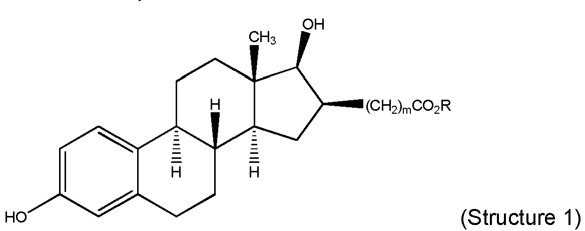 où m vaut 0, 1, ou 2 et R est H, un groupe alkyle en C1 à C5, un vinyle, CF₃, CH₂CH₂F, CH₂CHF₂, ou CH₂CF₃, les composés suivants étant exclus du champ de la Structure 1 :

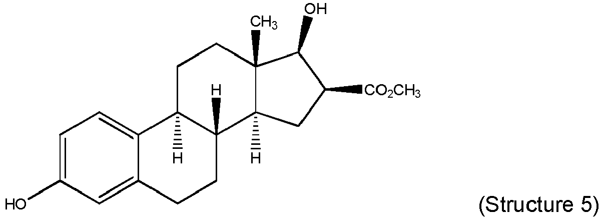

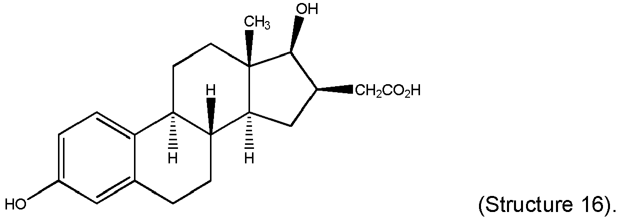

2. Le composé de la revendication 1, où m vaut 0 et R est un méthyle, un éthyle, un propyle, un iso-propyle, un butyle, un iso-butyle, un pentyle, un néo-pentyle, un vinyle, CF₃, CH₂CH₂F, CH₂CHF₂, ou CH₂CF₃.

3. Le composé de la revendication 1, choisi dans le groupe constitué par :

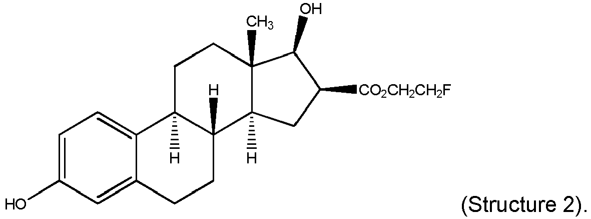

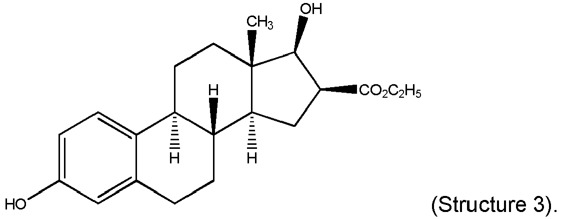

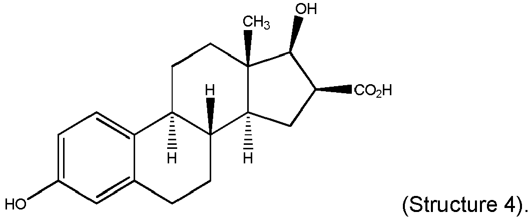

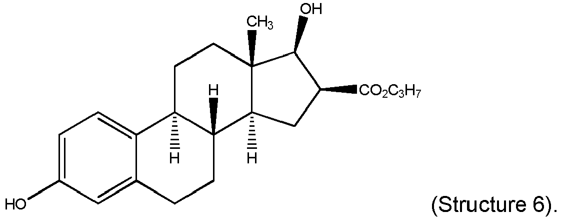

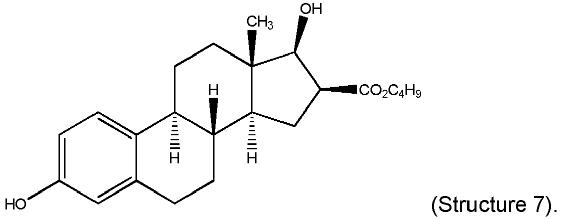

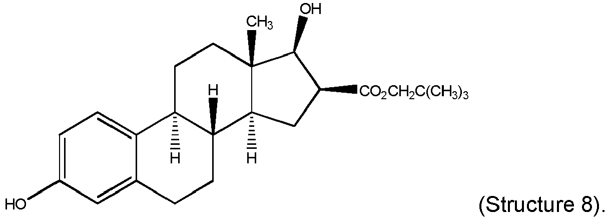

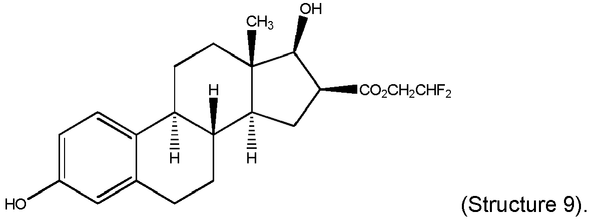

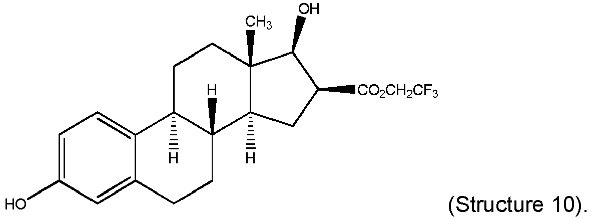

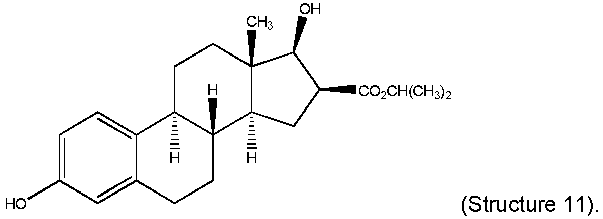

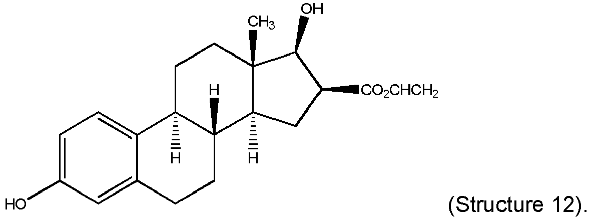

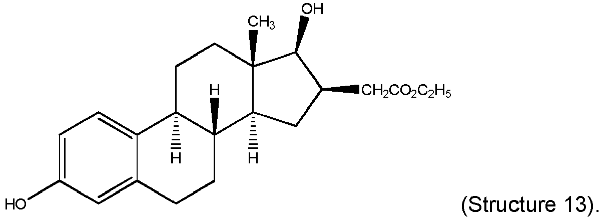

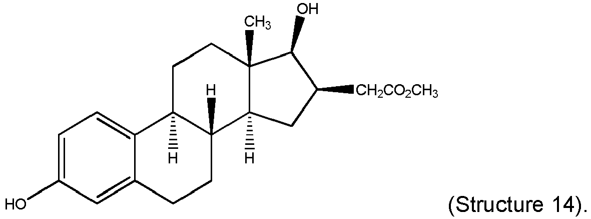

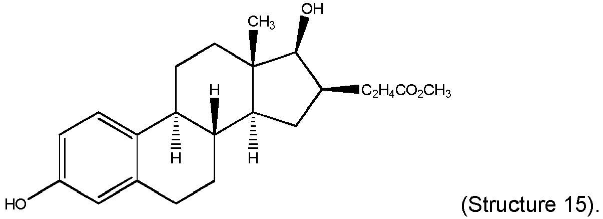

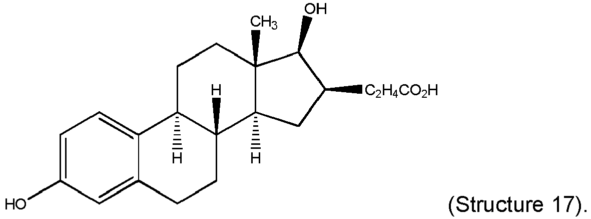

4. Le composé de la revendication 1, le composé n'étant pas a) la Structure 4, ou la Structure 17 (où R = H) ou b) la Structure 13 où ces composés sont tels que définis dans la revendication 3.

5. Le composé de la revendication 1, le composé étant un ester d'acide carboxylique et de 16β-œstradiol.

6. Une composition comprenant un composé substantiellement énantiomériquement pur selon l'une quelconque des revendications 1 à 5.

7. Une composition comprenant un composé selon l'une quelconque des revendications à 5, qui est substantiellement exempte d'autres composés oestrogéniques.

8. Une composition comprenant une quantité efficace d'un composé selon l'une quelconque des revendications 1 à 5 et un véhicule, un diluant, ou un excipient physiologiquement acceptable, formulé pour une administration topique.

9. La composition de la revendication 8, la composition étant formulée pour une administration topique sur la peau.

10. La composition de la revendication 8, la composition étant formulée pour une administration topique sur une membrane muqueuse.

11. La composition de la revendication 8, comprenant un composé substantiellement énantiomériquement pur selon l'une quelconque des revendications 1 à 5.

12. La composition de la revendication 8, la composition étant substantiellement exempte d'autres composés oestrogéniques.

13. La composition de n'importe lesquelles des revendications 6 à 12, la composition étant un cosmétique ou un cosméceutique.

14. Le composé de n'importe lesquelles des revendications 1 à 5 ou la composition de n'importe lesquelles des revendications 6 à 13 pour une utilisation en tant que médicament.

15. Le composé ou la composition de la revendication 14, où le médicament est destiné à une utilisation dans le traitement d'un sujet ayant une maladie ou un problème médical associé à une carence ou à un déséquilibre en oestrogènes.

16. Le composé ou la composition de la revendication 15, où la carence ou le déséquilibre en oestrogènes est dû à des androgènes non opposés.

17. Le composé ou la composition de la revendication 15, où la maladie ou le problème médical est relatif à la pousse des poils.

18. Le composé ou la composition de la revendication 17, où le sujet est une femme.

19. Le composé ou la composition de la revendication 18, où la femme est post-ménopausée.

20. Le composé ou la composition de la revendication 18, où la femme est pré- ou péri-ménopausée.
